# EUROPEAN PATENT APPLICATION

(11) **EP 4 821 452 A1**
(43) Date of publication of application: **14.10.2026**
(21) Application number: 25168752.1
(22) Date of filing: 07.04.2025
(51) Int. Cl.: A61B 18/04, A61B 18/08, A61B 18/14

(54) **MEDICAL DEVICE HAVING A COMPRESSIBLE HEATING ELEMENT**

(71) Applicant: Koninklijke Philips N.V., 5656 AE Eindhoven (NL)
(72) Inventor: MILAITIS, Maxwell, Eindhoven (NL); HOFFMANN, Julia, Eindhoven (NL); SHULEPOV, Sergey Yuryevich, Eindhoven (NL)
(74) Representative: Philips Intellectual Property & Standards

(57) **Abstract**

A medical device and its method of use are disclosed. The medical device includes a heating element adapted to be disposed in an elastic balloon. The heating element being adapted to be heated to a temperature to cause reversible denaturation of collagen in a vein.

## Description

### BACKGROUND

Collagen formation in vessel can cause occlusion and interfere or restrict blood flow through the vessel, causing burden on the cardiovascular system, and restriction of blood flow to certain body areas.

One type of collagen formation in vessels is Post Thrombotic Syndrome (PTS). In late PTS the thrombus forms into collagen which is a much tougher and more fibrous material. This collagen attaches to the endothelium and is very similar in properties to the venous wall, which is also largely composed of collagen. Due to the similarities, it is difficult to distinguish the two materials in the vessel. Therefore, methods like differential sanding and others that take advantage of the different material types to clear a vessel are dangerous and ineffective. Current thrombectomy devices use aspiration or maceration but these methods are ineffective for PTS since they are designed for softer material, blood clot. Many mechanical methods to remove disease states in the vessel can cause damage to the surrounding vessel. This damage leads to dysfunctional vessels and a clotting response which must be controlled by blood thinners.

What is needed, therefore, is a medical device that enables therapeutic procedures and measurement of blood flow that overcomes at least the noted drawbacks of the known approaches described above.

### SUMMARY

In accordance with a representative embodiment, a medical device or medical system is disclosed. The medical device or medical system comprises a heating element which, respectively, is disposed, or adapted to be disposed, in an elastic balloon. The heating element is adapted to be heated to a temperature to cause reversible denaturation of collagen in a vein. The elastic balloon may be part of the medical device and of the medical system. The elastic balloon may be disposed at, or adapted to be disposed at, a distal end of a catheter shaft comprised within the medical device and medical system.

In accordance with another representative embodiment, a method of carrying out a medical procedure is disclosed. The method comprises: providing a heating element in an elastic balloon; providing a fluid into the elastic balloon; and causing the heating element to expand when the elastic balloon is inflated causing reversible denaturation of collagen in a vein.

### BRIEF DESCRIPTION OF THE DRAWINGS

The example embodiments are best understood from the following detailed description when read with the accompanying drawing Figures. It is emphasized that the various features are not necessarily drawn to scale. In fact, the dimensions may be arbitrarily increased or decreased for clarity of discussion. Wherever applicable and practical, like reference numerals refer to like elements.
Fig. 1 is a perspective view of a medical device comprising a heating element in accordance with a representative embodiment.
Fig. 2A is a partial cross-sectional view of a heating element disposed in an elastic balloon in accordance with a representative embodiment.
Fig. 2B is a perspective view of a heating element in accordance with a representative embodiment.
Fig. 2C is a partial cross-sectional view of a heating element disposed in an elastic balloon in accordance with a representative embodiment.
Fig. 2D is a perspective view of a heating element in accordance with a representative embodiment.
Fig. 3A shows a temperature distribution of a medical device comprising a heating element in accordance with a representative embodiment.
Fig. 3B is a graph showing the impact on temperature of a heating element disposed in an elastic balloon over time with respect to applied current to the heating element, in accordance with a representative embodiment.
Fig. 4A is a perspective view of a heating element comprising a plurality of helical heating elements in accordance with a representative embodiment.
Fig. 4B is a partial cross-sectional view of heating element of Fig. 4A disposed in an elastic balloon in accordance with a representative embodiment.
Fig. 5 is a perspective view of a ballon for use in connection with a heating element in accordance with a representative embodiment.
Fig. 6 is a flow-chart of a method of reverse denaturation of collagen in a vessel in accordance with a representative embodiment.

### DETAILED DESCRIPTION

In the following detailed description, for the purposes of explanation and not limitation, representative embodiments disclosing specific details are set forth in order to provide a thorough understanding of embodiments according to the present teachings. However, other embodiments consistent with the present disclosure that depart from specific details disclosed herein remain within the scope of the appended claims. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. Definitions and explanations for terms herein are in addition to the technical and scientific meanings of the terms as commonly understood and accepted in the technical field of the present teachings.

It will be understood that, although the terms first, second, third etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

As used in the specification and appended claims, the singular forms of terms 'a', 'an' and 'the' are intended to include both singular and plural forms, unless the context clearly dictates otherwise. Additionally, the terms "comprises," and/or "comprising," and/or similar terms when used in this specification, specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to", "coupled to", or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" or "immediately adjacent" to another element or component, this encompasses only cases where the two elements or components are connected or disposed immediately adjacent to each other without any intermediate or intervening elements or components.

As used herein, an "elastic balloon" means the balloon is a compliant or semi-compliant balloon. Generally, percutaneous transluminal coronary angioplasty (PTCA) balloons are segmented based on a level of compliance in conforming to the shape of a vessel lumen. These subdivisions include compliant, non-compliant and semi-compliant balloons. Semi-compliant PTCA balloons are used in pre-dilatation of the lesion before the stent implantation. The non-compliant balloon catheters are usually ultra-high strength to handle the overinflation pressures to break up calcified lesions and to expend stents. Compliant balloons are comparatively low pressure and conform to the lumen size. Notably, non-compliant balloons are not contemplated by the present teachings.

As described herein in connection with various representative embodiments, the present teachings relate generally to a medical system, and a medical device and its method of use for reversible denaturation of collagen in vessels in a body. The medical device comprises a heating element adapted to be inserted into an elastic balloon and deployed near a lesion of a lumen in the body. In certain embodiments, the elastic balloon and heating element are expanded within a lesion and the heating element is activated. Fluid is provided in the elastic balloon, and the heating element is adapted to heat the fluid in the elastic balloon to a temperature that softens the collagen through reversible denaturation, resulting in destructuring of protein of the collagen through breaking of tertiary and quaternary protein structural bonds, softening the lesion. The elastic balloon applies outward (radial) force to the softened lesion to increase the size of the lumen. After reaching the desired shape, the heating element is turned off and balloon pressure is maintained while the fluid and surrounding tissue return to body temperature. The cooling of the fluid reverses the denaturation of the collagen, which now has a different shape (i.e., is stretched) due to the expansion caused by the elastic balloon. The collagen is no longer denatured, and the enlarged lumen is maintained. Among other applications, the system, device and method of the present teachings may be used in the peripheral venous system for the treatment of post thrombotic syndrome (PTS).

Among other clear improvements to the technical field of medical treatment, the medical systems and devices of the various representative embodiments include rearrangement of collagen fibers by reversible denaturation resulting in improved blood flow by non-invasive treatment, and a reduction or elimination of the need for blood thinners. By contrast, certain known thrombectomy devices are not sufficient for this clotting stage. Aspiration thrombectomy devices do not work due to collagen's toughness and strong adhesion to the endothelial wall. The aspiration is not strong enough to pull the collagen from the wall and cannot differentiate collagen from endothelial wall. Other devices that mechanically remove thrombus from the vessel are similarly not strong enough, and without differentiation from the venous wall, can cause damage to the vessel. Other devices which have attempted to treat PTS may result in some collagen removal but cause great damage to the vessel wall. This damage promotes further clotting response which decreases the success of disease treatment. Finally, particulates produced by known mechanical devices are also problematic. By contrast, and as described in connection with various representative embodiments below, the medical devices of the present teachings avoid generation of particulates by rearranging collagen fibers by reversible denaturation, rather than by attempting to remove the collagen.

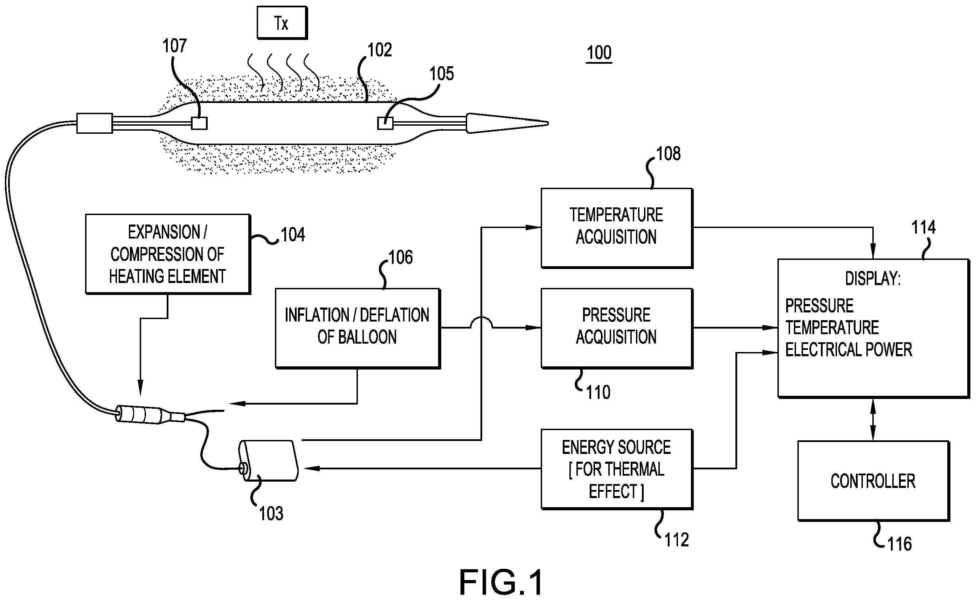

Fig. 1 is a perspective view of a medical system 100 for use with a medical device 102 comprising a heating element disposed in an elastic balloon in accordance with a representative embodiment. Various configurations of heating elements of various representative embodiments are described in further detail below. Notably, the medical device 102 depicts an illustrative embodiment comprising first and second electrodes 105, 107 disposed apart (e.g., at proximal and distal ends of the elastic balloon). In certain representative embodiments described more fully below, radio frequency (RF) radiation is transmitted between the electrodes from an RF source, resulting in heating of fluid (e.g., saline or a contrast agent) disposed in the elastic balloon. The heated fluid causes the denaturation of collagen in a lumen to carry out the procedure. In other representative embodiments described more fully below, heating elements are disposed along at least a portion of the length of the elastic balloon and direct current (DC) or alternating current (AC) is applied to the heating element resulting in heating of the fluid in the elastic balloon and denaturation of the collagen.

As described more fully below in connection with representative embodiments, the heating element (not shown in Fig. 1) is adapted to provide resistive heating, or heating with appropriate electromagnetic signals (e.g., RF or microwave signals) to heat the elastic balloon. Specifically, a source of electrical power (DC or AC), or a signal generator are provided as described more fully below.

In various representative embodiments, the heating element is disposed inside the elastic balloon, and may be coaxial with the elastic balloon. The heating element is compressed for insertion and removal from/with the elastic balloon and expanded in conjunction with the elastic balloon's inflation (expansion) and deflation. In the inflated state, the heating element is in its expanded state and current will be sent through, producing heat. In certain representative embodiments, the (resistive) heating element will continue to increase in temperature until the elastic balloon's exterior reaches the targeted temperature to rearrange the collagen. After treatment, the heating element will be compressed, and the elastic balloon will be deflated to facilitate removal of the catheter.

The medical system 100 comprises a temperature acquisition device (e.g., a thermistor) 108 disposed adjacent to the medical device to monitor the temperature of and along the length of the medical device. As described more fully below, the medical device 102 is heated to a desired temperature suitable to effect denaturation, but low enough to avoid tissue damage to the portion of the body in which the medical device is deployed.

The medical system 100 further comprises a pressure acquisition device 110 adapted to monitor the pressure applied to the lumen during a treatment. As described more fully below, the pressure acquisition device 110 is connected to a pressure source 106 adapted to provide fluid to the medical device and maintain a certain pressure in the medical device 102. Generally, pressure is applied by a known technique, such as by hand, and is measured with a known pressure meter.

The medical system 100 further comprises an energy source 112 connected to an input 103 and adapted to effect the heating in the fluid of the medical device 102. As described in connection with various representative embodiments, the energy source 112 may be a DC or an AC electrical source adapted to provide current to the heating element (not shown in Fig. 1), resulting in resistive heating of the fluid. Alternatively, the energy source may be an RF source adapted to transmit RF signals between first and second electrodes 105, 107 to heat the fluid in the elastic balloon.

The medical system 100 further comprises a user interface 114 comprising a display. As described more fully below, the user interface 114 is configurable to enable heating of the fluid in the medical device 102 to a suitable temperature to carry out the reversible denaturation of collagen in the lumen in which the medical device is deployed. As described more fully below, the user interface 114 enables the user to set the desired pressure and temperature of the fluid of the medical device 102 and to monitor the electrical power applied to the heating element during the procedure. Moreover, the user interface 114 provides real-time data from the procedure including temperature and pressure monitoring and applied electrical power. As described more fully below, the applied electrical power and resulting temperature, and the pressure applied to the medical instrument during the reversible denaturation process are monitored to carefully control the temperature and pressure during the medical procedure.

The medical system 100 further comprises a controller 116 adapted to provide control signals to the provide a desired set of power, temperature and pressure to the medical device to carry out the medical procedure. As described more fully herein, based on inputs to the user interface 114, the controller 116 sets the electrical power applied to the heating element of the medical device 102. Moreover, the controller 116 provides fluid at a desired pressure via the pressure source 106. Furthermore, the temperature acquisition device 108 may be useful in providing feedback to the controller 116 to control the active elements (e.g., heating element) disposed in the elastic balloon to avoid overheating of the fluid in the balloon 104.

The controller 116 may be implemented by a computer that includes more elements than the controller of Fig. 1B. Notably, in accordance with a representative embodiment, the controller may be remote to the medical system 100, and is adapted to control various aspects of the medical system 100 remotely via connections including both wired and wireless connections and protocols. In this sense, the controller 116, at least in part is a specialty or particular computer useful in controlling the medical system 100.

The controller 116 may operate as a standalone device or may be connected, for example, using a network to other computer systems or peripheral devices. In representative embodiments, the medical system 100 performs logical processing based on digital signals received via an analog-to-digital converter. The controller 116 can also be implemented as or incorporated into various devices, such as a workstation that includes a controller, a stationary computer, a mobile computer, a personal computer (PC), a laptop computer, a tablet computer, or any other machine capable of executing a set of software instructions (sequential or otherwise) that specify actions to be taken by that machine. The controller 116 can be incorporated as or in a device that in turn is in an integrated system that includes additional devices. In an embodiment, the controller 116 can be implemented in a device that also provides video or data communication. Moreover, the controller 116 may be connected to components of the system via a local wired interface such as an Ethernet cable or via a local wireless interface such as a Wi-Fi connection.

The processor may be considered a representative example of a processor of the controller 116 and executes instructions to implement some or all aspects of methods and processes described herein. The processor is tangible and non-transitory. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The processor is an article of manufacture and/or a machine component. The processor is configured to execute software instructions to perform functions as described in the various embodiments herein. The processor may be a general-purpose processor or may be part of an application specific integrated circuit (ASIC). The processor may also be a microprocessor, a microcomputer, a processor chip, a controller, a microcontroller, a digital signal processor (DSP), a state machine, or a programmable logic device. The processor may also be a logical circuit, including a programmable gate array (PGA), such as a field programmable gate array (FPGA), or another type of circuit that includes discrete gate and/or transistor logic. The processor may be a central processing unit (CPU), a graphics processing unit (GPU), or both. Additionally, any processor described herein may include multiple processors, parallel processors, or both. Multiple processors may be included in, or coupled to, a single device or multiple devices.

The term "processor" as used herein encompasses an electronic component able to execute a program or machine executable instruction. References to a processor should be interpreted to include more than one processor or processing core, as in a multi-core processor. A processor may also refer to a collection of processors within a single computer system or distributed among multiple computer systems.

The memory may include a main memory and/or a static memory, where memories in the medical system 100 communicate with each other and the processor via a bus (not shown). The memory may be considered a representative example of a memory of the controller 116, and store instructions used to implement some or all aspects of methods and processes described herein. Memories described herein are tangible storage mediums for storing data and executable software instructions and are non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The memory is an article of manufacture and/or machine components. The memory is a computer-readable medium from which data and executable software instructions can be read by a computer (e.g., by the processor of the controller 116). The memory may be implemented as one or more of random-access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM), digital versatile disk (DVD), floppy disk, Blu-ray disk, or any other form of storage medium known in the art. The memory may be volatile or non-volatile, secure and/or encrypted, unsecure and/or unencrypted. The inventive concepts also encompass a computer readable medium that stores instructions that cause a data processing system (such as the DSP of an NVA) to execute the methods described herein. Finally, a computer readable medium is defined herein to be any medium that constitutes patentable subject matter under 35 U.S.C. §101 and excludes any medium that does not constitute patentable subject matter under 35 U.S.C. §101. Examples of such media include non-transitory media such as computer memory devices that store information in a format that is readable by a computer or data processing system. More specific examples of non-transitory media include computer disks and non-volatile memories.

Additionally, the memory is an example of a computer-readable storage medium. Computer memory is any memory which is directly accessible to a processor. Examples of computer memory include, but are not limited to random access memory (RAM), registers, and register files. References to "memory" should be interpreted as possibly being multiple memories. The memory may for instance be multiple memories within the same computer system. The memory may also be multiple memories distributed amongst multiple computer systems or computing devices. Software instructions, when executed by the processor, perform one or more steps of the methods and processes as described herein. In an embodiment, the software instructions may reside all or in part within the memory and/or the processor during execution by the controller 116.

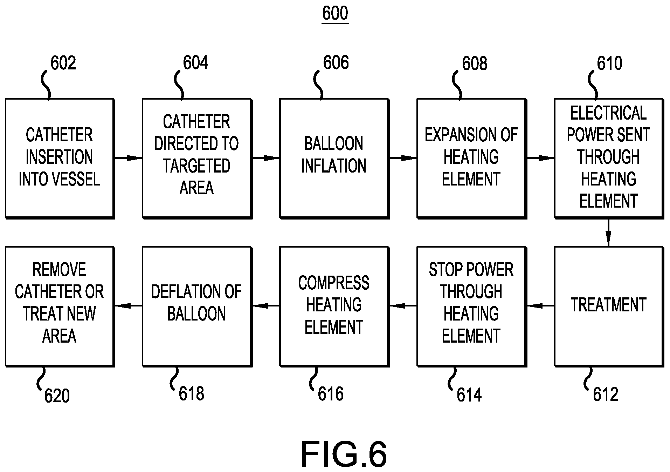

Commands from the controller 116 based on the selections at the user interface 114, along with manual manipulation of the elements of the medical device 102 (e.g., expanding and collapsing heating elements of various representative embodiments), enable the reversible denaturation of the collagen resulting in restructuring of the collagen (and thus the lesion) and opening the lumen in a diseased region. An illustrative sequence of carrying out the medical procedure is described in further detail in connection with Fig. 6 below.

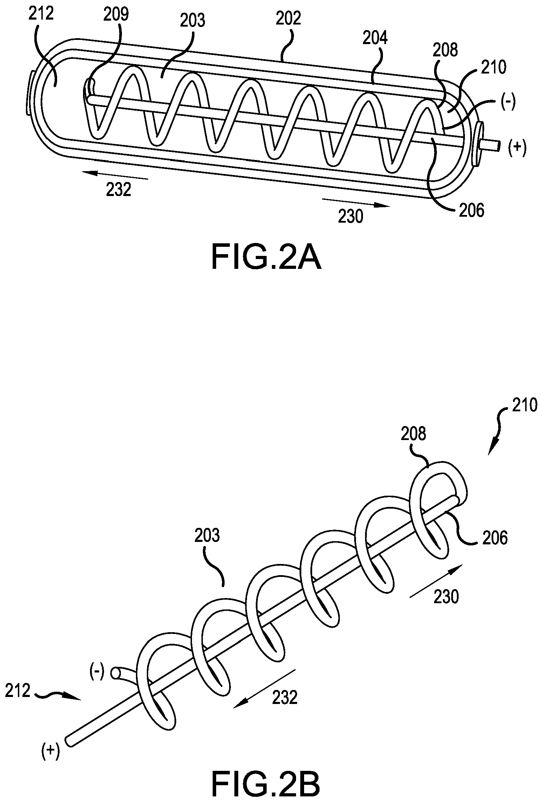

Fig. 2A is a partial cross-sectional view of a medical device 202 comprising a heating element 203 disposed in an elastic balloon 204 in accordance with a representative embodiment. Various aspects of the medical system and medical device described in connection with Fig. 1 are common to the presently described representative embodiments, and may not be repeated to avoid obscuring the presently described representative embodiments.

The heating element 203 comprises a central heating element 206 and a helical heating element 208. Notably, a catheter lumen and a guide wire are not shown in Fig. 2A to enable clear description of the components of the heating element 203. The helical heating element 208 makes a first electrical contact at a terminal (e.g., positive terminal (not shown)) in a power supply (not shown) of the medical device 202, and a second electrical contact 209 at a second end 212 (e.g., a distal end) of the heating element 203. In the present representative embodiment, the heating element 203 comprises a resistive material so current is applied between the first and second electrical contacts of the power supply, the heating element 203 resistively heats to provide heat to fluid (e.g., saline or contrast agent, or other suitable material to provide a heat medium for the heating element) to carry out the denaturation of collagen in the lumen in which the medical device is disposed. As described more fully below, after the elastic balloon 204 reaches a desired temperature to cause denaturation, the heating element is cooled passively or actively (e.g., with a cooling fluid) to reverse the denaturation resulting in the restructuring of the collagen in a way that reduces the blockage caused by the collagen in the lumen.

As described more fully below, the heating element 203 is expanded by applying force to the helical heating element 208 in a direction 232, and is collapsed by applying force to the helical heating element 208 in another direction 230 (see Fig. 2A). Accordingly, expansion of the heating element by moving the helical heating element 208 in the direction 232 can be carried out after its insertion in the collapsed state by simple manual manipulation of the helical heating element 208 by the user.

As described more fully below, in certain embodiments, the elastic balloon 204 is expanded to a desired degree to treat the lesion by introducing fluid to the elastic balloon 204, with the helical heating element 208 expanded after the elastic balloon 204 is expanded. In other representative embodiments, the helical heating element 208 is expanded before or during introduction of fluid to expand the elastic balloon 204 to the desired degree to treat the lesion.

Fig. 2B is a perspective view of a heating element 203 in accordance with a representative embodiment. Various aspects and details of the heating element 203 are common to those described in connection with the representative embodiments of Figs. 1-2A, and may not be repeated to avoid obscuring the presently described representative embodiments.

In accordance with representative embodiments, the heating element 203 comprises a shape memory alloy (SMA) suitable for use in medical devices (e.g., Nitinol) that is heat set in the expanded state shown in Fig. 2B. As will be appreciated more fully as the present description continues, in certain representative embodiments, the temperature for the heat setting of the shape memory alloy of the heating element 203 in its expanded states is approximately equal to a desired temperature (e.g., 42°C) to effect the denaturation of the collagen of the lesion. In this way, the helical heating element 208 of heating element 203 is in its expanded state, and upon application of current in the electrical circuit comprising the heating element 203, fluid (not shown) in the elastic balloon 204 heats the collagen to the desired temperature to effect denaturation when the elastic balloon is in the proper expanded state to result in the desired restructuring of the collagen in the lesion to open the lumen to a desirable degree. Moreover, and as described more fully below, the temperature of the medical device 202 is substantially uniform across its length, and at the desired temperature. This results in effective reversible denaturation of the collagen of the lumen in which the medical device is disposed, and reduces, if not eliminates, overheating (e.g., hot spots) that can have a deleterious effect (e.g., overheating/scarring the tissue of the lumen), or underheating that can result in insufficient denaturation of the collagen in regions that are not heated to the proper temperature.

It is noted that the use of shape memory alloy for the heating element is not essential, and other materials may be selected to provide the resistive heating of the heating element for proper denaturation of the collagen to restructure the lesion. These materials include, but are not limited to, stainless steel or other suitable material for medical use according to the present teachings.. Furthermore, the diameter of the wire used for the central heating element 206 and the helical heating element 208 are in the range of approximately 0.5 mm to approximately 2.0 mm, and is determined based on a number of factors including resistance (the resistance of the wire is inversely proportional to the diameter of the wire), strength and compatibility.

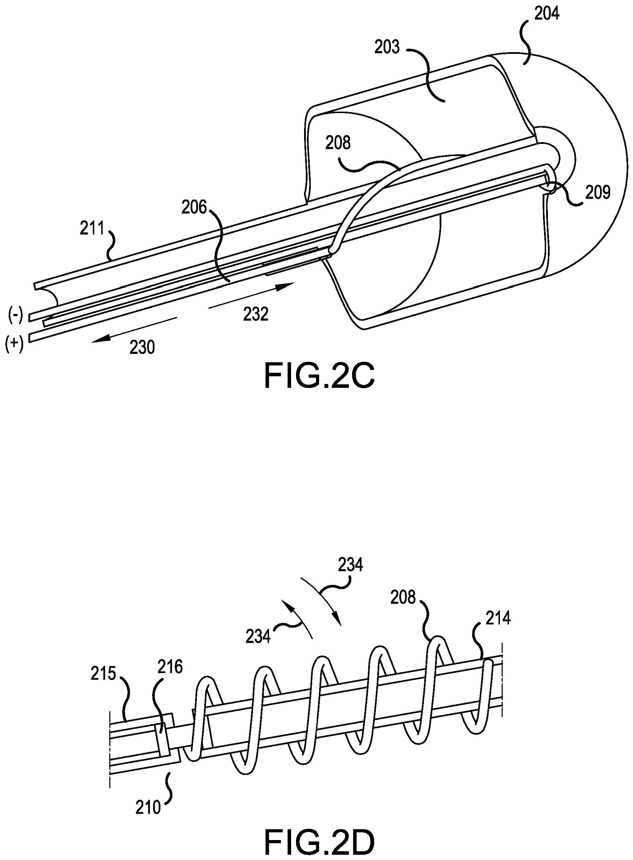

Fig. 2C is a partial cross-sectional view of a heating element disposed in an elastic balloon 204 in accordance with a representative embodiment. Various aspects and details of the heating element 203 are common to those described in connection with the representative embodiments of Figs. 1-2B, and may not be repeated to avoid obscuring the presently described representative embodiments.

As shown, the central heating element 206 is disposed in a catheter lumen 211, and the helical heating element 208 is disposed around the catheter lumen 211. Again, the helical heating element 208 makes electrical contact (positive contact) at the power supply (not shown) and at the second electrical contact 209 to complete the electrical circuit.

The heating element 203 is shown in Fig. 2C in its compressed (collapsed) state. Notably, after being in its inflated/expanded state as shown in Figs. 2A-2B, the heating element is collapsed for removal. Similarly, the heating element 203 is in the compressed state for insertion at the beginning of the procedure.

As noted above, pulling applying force to the helical heating element 208 in the direction 230 results in collapse of the helical heating element 208 enabling relatively simple manual manipulation of the central heating element 206 by the user to collapse the helical heating element 208 for removal. By contrast, applying force to the helical heating element 208 in the direction 232 expands the helical heating element 208 for heating the fluid in the elastic balloon 204. As alluded to above, once collapsed, the helical heating element 208 allows for the removal of the heating element 203, or its movement to another location where a treatment is desired. After the heating element 203 is located at the other location, applying force in the direction 232 expands the helical heating element 208 for heating the fluid and treatment at the other location.

Fig. 2D is a perspective view of a heating element 203 in accordance with a representative embodiment. Various aspects and details of the heating element 203 are common to those described in connection with the representative embodiments of Figs. 1-2C, and may not be repeated to avoid obscuring the presently described representative embodiments.

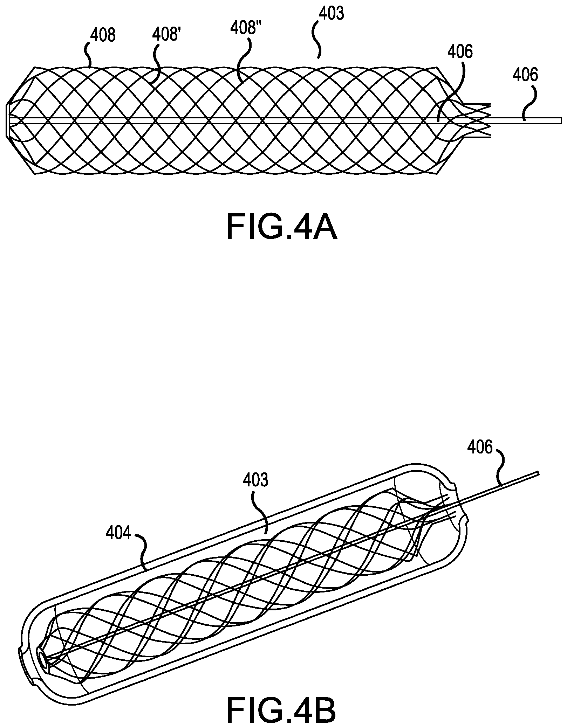

The heating element 203 is expanded by rotation of a shaft 216 connected to a rod 214 in a direction 234, and is compressed by rotation of the rotational element 214 in another direction 236. The first end 210 of the helical heating element 208 is attached to a catheter 215, which in turn is connected to one terminal of the power supply (not shown). The opposing end of the helical heating element 208 is connected to the rotational element 214, which in turn is connected to the other terminal of the power supply. As such, when the heating element 203 is rotated in one direction 234, the diameter of the helical heating element 208 increases in order to ensure proper heating of the elastic balloon (not shown in Fig. 2D) to a desired temperature. Similarly, rotation in the direction 236 causes the diameter of the helical heating element 208 to decrease, allowing for the elastic balloon to collapse. Notably, this method of expanding and collapsing the helical heating element for heating and insertion and removal of the heating element 203 can also be done with heating elements comprising a plurality of helices, such as shown and described in connection with Figs. 4A-4B. Finally, while shape memory alloy may be used for the helical heating element 208, because the expansion/contraction of the helical heating element 208 may be caused by rotation of the rotational element 214, other suitable materials such as noted above may be used.

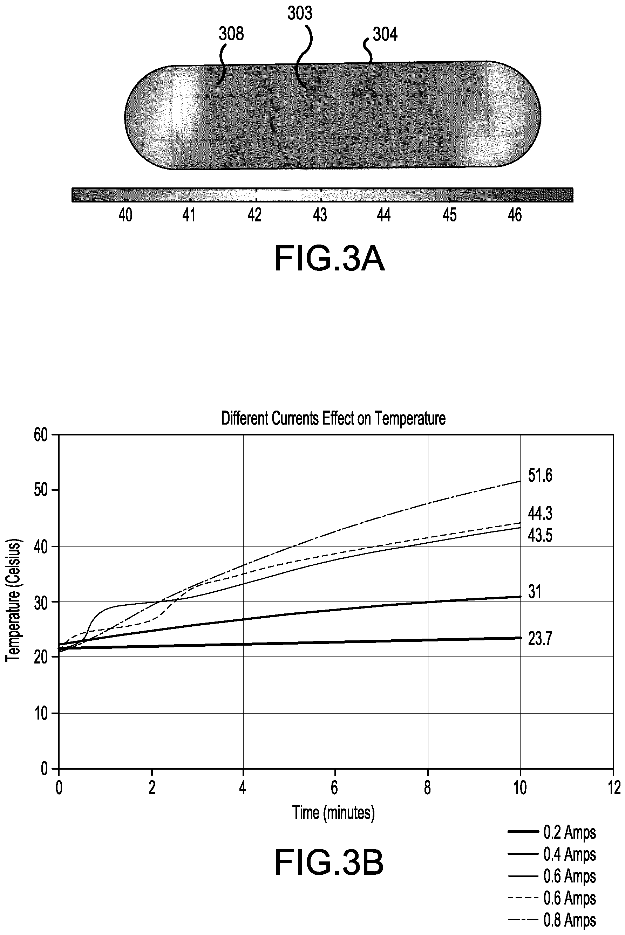

Fig. 3A shows a temperature distribution of a medical device 302 comprising a heating element disposed in an elastic balloon 304 in accordance with a representative embodiment. Various aspects and details of the medical device 302 are common to those described in connection with the representative embodiments of Figs. 1-2D, and may not be repeated to avoid obscuring the presently described representative embodiments.

As shown in Fig. 3A, the medical device 302 comprises the elastic balloon 304 and the heating element comprising a helical heating element 308 in an expanded state. Specifically, and as alluded to above, fluid is provided in the elastic balloon 304 to provide a heat medium in the medical device.

As can be seen from a review of Fig. 3A, the temperature on the exterior surface of the elastic balloon 304 across a significant portion of the medical device 302 is substantially uniform. As alluded to above and described more fully below, the elastic balloon 304 is inflated/expanded with fluid, and the heating element including the helical heating element 308 is also in an expanded state (not visible in Fig. 3A)). Accordingly, the components of the medical device 302 are set to effect heating of the fluid in the elastic balloon 304 using the heating element, and as shown, provide the substantially uniform temperature across a significant length of the medical device. Accordingly, with the medical device 302 at the desired temperature for denaturation of the collagen of the lesion in the lumen, followed by cooling to restructure the collagen to open the lumen for better fluid (e.g., blood) flow. Moreover, the heat uniformity and the temperature stability of the medical device 302 enable the heating to the desired temperature for denaturation of the collagen without significant hot spots that could cause damage to the tissue of the lumen as noted above.

The substantially uniform temperature and temperature stability of the medical device 302 is realized by the selection of not only a proper material for the components of the heating element and the size (diameter/gauge) of the wires that form the heating element, but also the number of turns in the helical heating element 308 of the heating element. As described above, the helical heating element 308 of the heating element 303 may comprise a shape memory alloy with its heat setting being at a temperature near the desired temperature to carry out the denaturation. Again, although this is not essential. Notably, other materials may be selected to provide the resistive heating of the heating element for proper denaturation of the collagen to restructure the lesion. These materials include, but are not limited to, stainless steel or other suitable material for medical use according to the present teachings. Furthermore, the diameter of the wire used for the rod (not visible in Fig. 3A) and the helical heating element 308 are in the range of approximately 0.5 mm to approximately 2.0 mm, and is determined based on a number of factors including resistance (the electrical resistance of the wire is inversely proportional to the diameter of the wire), strength and compatibility.

As noted above, the heating element 303 is heated by resistive heating due to current flow through the wires in the heating element. In certain representative embodiments, a DC power supply is applied to the heating element to cause resistive heating of the components of the heating element. The application of DC power to the heating element heats the fluid in a relatively short duration of time and in a uniform manner. However, regulations governing the current levels that may be applied to the medical device for heating are rather strict. Accordingly, in other representative embodiments, the heating element provides resistive heat by applying AC power to the heating element. Among other benefits, providing power to the heating element from an AC power supply, less power is needed to heat the fluid in the medical device uniformly to the desired temperature, resulting in a safer medical device, and one that is more acceptable in view of regulations governing the specifications of the medical device 302. Furthermore, resistive heating of the heating element to the desired temperature by AC power is also found to be faster, presumably because of comparatively improved transmission of heat in the fluid that is realized by the application of AC power. Moreover, a more uniform temperature distribution may also be realized by use of AC power to the heating element.

A third source of electrical power is electromagnetic energy at specific frequencies, including microwave and RF frequencies. In one representative embodiment, RF power is applied to the heating element, which causes mechanical vibrations of the components of the heating element. These mechanical vibrations result in resonance in the fluid (e.g., saline), and faster heating times with comparatively low power. Alternatively, as noted above, the RF or microwave signals may be applied between two electrodes disposed in the proximal and distal ends of the elastic balloon. This results in a resonance condition of the water molecules in the saline solution, for example, and compared to DC resistive heating, ultimately may improve the speed of heating of the fluid to the desired temperature to effect the denaturation of the collagen, while maintaining the desired uniform temperature along the length of the medical device 302.

Finally, regardless of the source of electrical power, after the medical device reaches the desired temperature for a desired period of time to effect denaturation of the collagen with the elastic balloon in an expanded state, the source of electrical power is turned off. This allows for the fluid to cool resulting in more tertiary and quaternary structural bonds to be formed within collagen in the lumen (denaturation is reversed), with the expanded balloon's "moving" the collagen to a restructured state, and enlarging the opening of the vessel that was compromised by the lesion before performing the temporary denaturation procedure.

Fig. 3B is a graph showing the impact on temperature of a heating element disposed in an elastic balloon over time and applying a plurality of current to the heating element, in accordance with a representative embodiment. Various aspects and details of the medical device 302 are common to those described in connection with the representative embodiments of Figs. 1-3A, and may not be repeated to avoid obscuring the presently described representative embodiments.

Referring to Fig. 3B, the time to reach a certain temperature for plurality of currents applied to the heating element of medical device 302 are shown. As will be appreciated, increasing the current to the wires of the heating element reduces the time required to reach a certain temperature. Moreover, in a representative embodiment, if power is applied to the heating element for 20 minutes, in some cases, the temperature at the exterior of the elastic balloon 304 does not reach a desired temperature near 40°C, whereas some currents applied to the wires for 20 minutes will result in heating the fluid in the elastic balloon 304 to a temperature that may not be safe (e.g., 0.8A for 20 min reaches 51.6 °C).

Data such as those provided in Fig. 3B allows the user to apply the minimum current to reach a target temperature in a reasonable time. Just by way of illustration, a target temperature of approximately of 42°C is useful for reversible denaturation of the collagen in the lumen in approximately 10 min. In this way, denaturation of the collagen softens it and allows its shape to be altered (i.e., is stretched) before cooling. Once the lumen is expanded to a desirable size, denaturation is reversed and the lumen is maintained so as to reduce flow resistance/blockage of the lumen as described herein.

In a representative embodiment, controller 116 may be programmed to set the temperature and time requirement via the user interface 114 to enable the procedure to be carried out.

As noted above, the temperature may be realized using a thermistor adjacent to the exterior of the elastic balloon, for example. In another representative embodiment, the actual change in resistance of the wires caused by joule heating is used. The controller 116 is adapted to use the wire's resistance change to predict the temperature at that resistance based on a resistance curve of the materials of the wire to provide the desired temperature. By having a constant measure of the wire's resistance, the change in resistance due to temperature is used to gain a temperature reading. The temperature may be measured using a Wheatstone bridge after activation of the heating element. The controller 116 uses feedback from the Wheatstone bridge to control the power output to the heating element to maintain a uniform temperature on the exterior of the elastic balloon 304. The temperature may be provided on the display of the user interface to the operator to assist proper use of medical device 302.

Fig. 4A is a perspective view of a heating element 403 comprising a plurality of helical heating elements 408, 408', 408" connected to a central heating element 406 in accordance with a representative embodiment. Various aspects and details of the heating element 403 are common to those described in connection with the representative embodiments of Figs. 1-3B, and may not be repeated to avoid obscuring the presently described representative embodiments.

In various representative embodiments, some or all of the plurality of helical heating elements 408, 408', 408" may be made of a suitable shape memory alloy with its heat setting in an expanded state at or near the desired temperature to carry out the denaturation procedure. It is noted that the use of shape memory alloy for the helical heating elements 408, 408', 408" is not essential, and other materials may be selected to provide the resistive heating of the heating element for proper denaturation of the collagen to restructure the lesion. These materials include, but are not limited to, stainless steel or other suitable material for medical use according to the present teachings. Furthermore, the diameter of the wire used for the central heating element 406 and the helical heating elements 408, 408', 408" are in the range of approximately 0.5 mm to approximately 2.0 mm, and is determined based on a number of factors including resistance (the resistance of the wire is inversely proportional to the diameter of the wire), strength and compatibility.

Although the function of the multiple helical heating elements 408, 408', 408" is substantially identical to the single helix elements of representative embodiments described above, the multiple helical heating elements 408, 408', 408" beneficially provide an increased surface area to heat the fluid (not shown in Fig. 4A). Among other advantages, the increased surface area results in faster, more uniform heating of the medical device in which the heating element 403 is deployed.

Fig. 4B is a partial cross-sectional view of a medical device 402 comprising the heating element of Fig. 4A disposed in an elastic balloon 404 in accordance with a representative embodiment. Various aspects and details of the heating element 403 are common to those described in connection with the representative embodiments of Figs. 1-4A, and may not be repeated to avoid obscuring the presently described representative embodiments.

As described above and below, in operation, the collapsed heating element 403 is disposed in the elastic balloon 404 for insertion of the medical device 402 into a lumen for treatment. Once the medical device 402 is deployed, fluid is introduced to the elastic balloon 404 causing its inflation. Moreover, in this embodiment, the expansion of the elastic balloon 404 causes the expansion of the multiple helical heating elements 408, 408', 408" resulting in the heating element being deployed in its expanded state. Application of current via the central heating element 406 results in heating of the fluid by the heating element 403 to perform denaturation of collagen in the lumen where the medical device is located. Furthermore, after reversing the denaturation of the collagen, which now has a different shape due (i.e., is stretched) to the expansion caused by the elastic balloon 404, the elastic balloon 404 is collapsed, which collapses the plurality of helical heating elements 408, 408', 408."

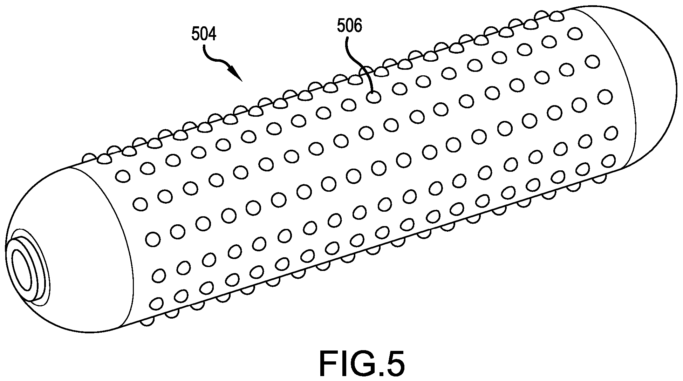

Fig. 5 is a perspective view of a textured elastic ballon 504 for use in connection with a heating element (not shown in Fig. 5) in accordance with a representative embodiment. Various aspects and details of the heating element 403 are common to those described in connection with the representative embodiments of Figs. 1-4B, and may not be repeated to avoid obscuring the presently described representative embodiments.

As shown, the textured elastic balloon 504 comprises a plurality of focal points 506. Upon expansion of the textured elastic balloon 504, the textured exterior provided by the focal points 506 focus stress on points of the interior wall of the lumen, increasing rearrangement of collagen. By increasing stress on focal points 506, the fatigue of the collagen can increase after heating the fluid/elastic balloon to the desired temperature. Specifically, the textured elastic balloon 504 causes depressions and elevations of the collagen in the lumen to increase stress during denaturation resulting in further stretching of the collagen to a desired shape to improve fluid flow through the lumen. The textured elastic balloon 504 also resists slipping within the vessel, so the desired location is treated.

Fig. 6 is a flow-chart of a method 600 of reversible denaturation of collagen in a lumen in accordance with a representative embodiment. Various aspects and details of the method are common to those described in connection with the representative embodiments of Figs. 1-5, and may not be repeated to avoid obscuring the presently described representative embodiments.

At 602, a catheter is inserted into a lumen by known technique.

At 604, the catheter is moved to a position in an area targeted for reversible denaturation to provide a different shape to (i.e., stretch) the collagen to improve fluid flow therethrough.

At 606, the method 600 continues with the inflation of the elastic balloon. As noted above, fluid is introduced to the elastic balloon to reach a desired inflation/expansion state.

At 608, the method 600 continues with expansion of the heating element as described more fully above in connection with various representative embodiments. Notably, in certain embodiments in which the heating element comprises a plurality of helical heating elements, the expansion of the heating element can be effected when the elastic balloon is inflated.

At 610, the method 600 continues with providing electrical power to the heating element of the medical device to a desired temperature. At 612, the fluid in the balloon is heated to a desired temperature so the outer surface of the elastic balloon is heated to a desired temperature for denaturation to be carried out.

At 614, the method 600 continues with the termination of applying electrical current to the heating element. This allows the fluid in the elastic balloon to cool and allows the denaturation to be reversed. Notably, in certain representative embodiments, this step may comprise introducing a cooling fluid into the elastic balloon to expedite the cooling process.

At 616, the elastic balloon is collapsed. As described in certain representative embodiments, the heating element may be collapsed before the elastic balloon is collapsed, whereas in other representative embodiments, the collapsing of the balloon collapses the heating element.

At 618, the method 600 ends with the removing of the catheter, or moving the medical device to another area for the reversible denaturation process to be repeated.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The Abstract of the Disclosure is provided to comply with 37 C.F.R. §1.72(b) and is submitted with the understanding that it will not be used to interpret or limit the scope or meaning of the claims. In addition, in the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the true spirit and scope of the present disclosure. Thus, to the maximum extent allowed by law, the scope of the present disclosure is to be determined by the broadest permissible interpretation of the following claims and their equivalents and shall not be restricted or limited by the foregoing detailed description.

According to additional embodiments , a medical device and its method of use are disclosed. The medical device includes a heating element adapted to be disposed in an elastic balloon. The heating element being adapted to be heated to a temperature to cause reversible denaturation of collagen in a vein.

Embodiment 1. A medical device, comprising:
a heating element adapted to be disposed in an elastic balloon, the heating element being adapted to be heated to a temperature to cause reversible denaturation of collagen in a vein.

Embodiment 2. The medical device of embodiment 1, wherein the heating element is adapted to collapse for removal from the elastic balloon, or for removal of the medical device from the vein.

Embodiment 3. The medical device of embodiment 1, wherein expansion of the heating element is adapted to cause the expansion of the elastic balloon.

Embodiment 4. The medical device of embodiment 1, wherein the heating element comprises a helical heating element, wherein helical heating element causes expansion and contraction of the heating element.

Embodiment 5. The medical device of embodiment 1, wherein the heating element comprises a plurality of helical heating elements, wherein movement of the plurality of helical heating elements cause expansion and contraction of the heating element.

Embodiment 6. The medical device of embodiment 1, wherein the heating element is connected to a power supply adapted to provide electrical power to the heating element.

Embodiment 7. The medical device of embodiment 6, wherein the electrical power is direct current (DC) electrical power to the heating element.

Embodiment 8. The medical device of embodiment 6, wherein the electrical power is alternating current (AC) electrical power to the heating element.

Embodiment 9. The medical device of embodiment 7, wherein the AC electrical power has a frequency in a radio frequency (RF) band.

Embodiment 10. The medical device of embodiment 1, wherein the heating element comprises a shape memory alloy having a transition temperature at a desired temperature for the reversible denaturation of the collagen.

Embodiment 11. The medical device of embodiment 10, wherein the transition temperature corresponds to an expanded state of the heating element at the desired temperature.

Embodiment 12. The medical device of embodiment 10, wherein the transition temperature corresponds to a collapsed state of the heating element at the desired temperature.

Embodiment 13. The medical device of embodiment 1, wherein, after expansion of the elastic balloon and applying heat through the heating element, a cooling fluid is added to the elastic balloon, and the collagen reverts to its consistency before heating.

Embodiment 14. The medical device of embodiment 13, further comprising providing a cooling fluid to the elastic balloon to facilitate cooling.

Embodiment 15. A method of carrying out a medical procedure, the method comprising:
providing a heating element in an elastic balloon;
providing a fluid into the elastic balloon; and
causing the heating element to expand when the elastic balloon is inflated causing reversible denaturation of collagen in a vein.

## Claims

1. A medical device, comprising:
a catheter shaft;
an elastic balloon disposed at a distal portion of the catheter shaft; and
a heating element disposed within the elastic balloon, the heating element being adapted to be heated to a temperature to cause reversible denaturation of collagen in a blood vessel.

2. The medical device of claim 1, wherein the heating element is adapted to radially expand and collapse within the elastic balloon.

3. The medical device of claim 1 or 2, wherein expansion of the heating element is adapted to cause the expansion of the elastic balloon.

4. The medical device of any one of claims 1 to 3, wherein the heating element comprises a helical heating element, wherein helical heating element causes expansion and contraction of the heating element.

5. The medical device of any one of claims 1 to 4, wherein the heating element comprises a plurality of helical heating elements, wherein movement of the plurality of helical heating elements cause expansion and contraction of the heating element.

6. The medical device of any one of claims 1 to 5, wherein the heating element is connected to a power supply adapted to provide electrical power to the heating element.

7. The medical device of claim 6, wherein the electrical power is direct current (DC) electrical power to the heating element.

8. The medical device of claim 6, wherein the electrical power is alternating current (AC) electrical power to the heating element.

9. The medical device of claim 8, wherein the AC electrical power has a frequency in a radio frequency (RF) band.

10. The medical device of any one of claims 1 to 9, wherein the heating element comprises a shape memory alloy having a transition temperature at a desired temperature for the reversible denaturation of the collagen.

11. The medical device of claim 10, wherein the transition temperature corresponds to an expanded state of the heating element at the desired temperature.

12. The medical device of claim 10, wherein the transition temperature corresponds to a collapsed state of the heating element at the desired temperature.

13. The medical device of any one of the claims 1 to 12, wherein the device is configured to:
expand the elastic balloon to an expanded state;
apply heat through the heating element; and
provide a cooling fluid to the elastic balloon to facilitate cooling of the collagen, to cause the collagen to revert to its consistency before heating.

14. A method of controlling a device as claimed in any one of the claims 1 to 14, the method comprising:
providing a fluid into the elastic balloon;
causing the heating element to expand when the elastic balloon is inflated; and
optionally, heat the heating element to a temperature for causing reversible denaturation of collagen in a vein.

15. A computer program comprising computer readable instructions which, when executed by a processor circuit, cause the method of claim 14 to be performed.

16. A medical system comprising:
a catheter shaft;
an elastic balloon for disposing at, or disposed at, a distal portion of the catheter shaft; and
a heating element configured to be disposed within the elastic balloon, the heating element being adapted to be heated to a temperature for causing reversible denaturation of collagen in a blood vessel.

17. The medical system of claim 16 wherein the system is for assembling into a medical device as claimed in any of the claims 1 to 13.
